# EUROPEAN PATENT APPLICATION

(11) **EP 4 119 103 A1**
(43) Date of publication of application: **18.01.2023**
(21) Application number: 21186272.7
(22) Date of filing: 16.07.2021
(51) Int. Cl.: A61F 2/44, A61F 2/46, A61F 2/30

(54) **ADJUSTABLE INTERVERTEBRAL CAGE, ASSOCIATED INSTRUMENT AND MANUFACTURING PROCESS THEREFOR**

(71) Applicant: Blue Ocean Spine GmbH, 78532 Tuttlingen (DE)
(72) Inventor: SALVERMOSER, Markus, 78532 Möhringen (DE); RICHTER, Jacob, 78532 Tuttlingen (DE); EISEN, Guntmar, 78532 Tuttlingen (DE)
(74) Representative: Stolmár & Partner Patentanwälte PartG mbB

(57) **Abstract**

The present invention discloses a process for manufacturing an adjustable intervertebral cage by 3D printing with a base plate, a moving plate, a height wedge and a lordosis wedge comprising the steps of: i) Providing a 3D printer with a substrate plate, and metal powder; ii) Applying metal powder to the substrate plate; iii) Selectively melting the metal powder on the substrate plate according to a layer of the adjustable intervertebral cage, wherein the layer of the base plate, the layer of the moving plate, the layer of the height wedge and the layer of the lordosis wedge are interlaced; and iv) Sequentially repeating steps ii) and iii) layer by layer until the adjustable intervertebral cage is fully manufactured, wherein all layers of the base plate, all layers of the moving plate, all layers of the height wedge and all layers of the lordosis wedge are each metallically bonded together.

## Description

### Field of the invention

The present invention relates to an adjustable intervertebral cage, an associated instrument and a manufacturing process therefor.

### State of the art

Adjustable intervertebral cages can be used to treat a variety of spinal disorders, including degenerative disc disease. The cages provide a space for inserting a bone graft material between adjacent vertebrae, and to promote fusion between the bones at this spine segment.

Cages can be implanted in several directions. One direction is from front to back or rather towards posterior. Corresponding surgical terms for this are PLIF (Posterior lumbar interbody fusion) and EPLIF (Extraforaminal posterior lumbar interbody fusion). Another implantation direction is from back to front or rather towards anterior. Corresponding surgical terms for this are ALIF (Anterior lumbar interbody fusion) and TLIF (Transforaminal lumbar interbody fusion). It is also possible to implant the cage from side to middle or rather towards medial. Corresponding surgical terms for this are LLIF (Lateral lumbar interbody fusion) and TLIF.

To ease insertion of the cage into the spine of the patient, the cage can be configured to be adjustable in height and/or in lordosis.

Such adjustable intervertebral cages according to the state of the art comprise many components to enable the adjustability of the cage. Additionally, every single component is manufactured separately which results in high manufacturing costs.

### Description of the invention

The problem underlying the present invention was therefore to provide a novel adjustable intervertebral cage, an associated instrument and a manufacturing process therefor enabling a high degree of adjustability and at the same time low manufacturing costs. This problem is solved by a process for manufacturing an adjustable intervertebral cage by 3D printing with a base plate, a moving plate, a height wedge and a lordosis wedge comprising the steps of:
i) Providing a 3D printer with a substrate plate, and metal powder;
ii) Applying metal powder to the substrate plate;
iii) Selectively melting the metal powder on the substrate plate according to a layer of the adjustable intervertebral cage, wherein the layer of the base plate, the layer of the moving plate, the layer of the height wedge and the layer of the lordosis wedge are interlaced; and
iv) Sequentially repeating steps ii) and iii) layer by layer until the adjustable intervertebral cage is fully manufactured, wherein all layers of the base plate, all layers of the moving plate, all layers of the height wedge and all layers of the lordosis wedge are each metallically bonded together.

Thus, an adjustable intervertebral cage can be manufactured at low costs.

The cages are preferably manufactured by Selective Laser Melting (SLM), e.g. with a 3D printer. Therefore, a substrate plate may be fastened to an indexing table inside a chamber with a controlled atmosphere of inert gas (e.g. argon or nitrogen).

Preferably, the metal powder is a titanium alloy, e.g. Ti-6AI-4V to enable biocompatibility.

Preferably, each 2D slice, or rather layer of the cage is fused by selectively melting the metal powder via a laser.

Preferably, the laser has enough energy to fully melt or rather weld the metal particles to form solid metal.

Preferably, before applying new metal powder, the substrate plate is lowered by the layer thickness (z-direction).

The completed part may be removed from the substrate plate by cutting or breaking off.

Preferably, the right amount of heat dissipation is ensured. If too less heat is dissipated before applying a new layer, exact geometry curves cannot be realized since newly applied metal powder is melted by the lower layer. If too much heat is dissipated before applying a new layer, the bond between the layers will be too weak, resulting in low component strength.

Since a new layer has to be connected to its lower layer, overhangs cannot be realized by SLM. The highest angle possible between 0° (parallel to the z-direction and directed towards the top of the cage) and 90° (perpendicular to the z-direction) is 45°. If angles above 45° are necessary, preferably supports are used.

The supports are preferably visible from outside of the cage so that they can be easily identified and removed after 3D printing.

Since all components of the cage are interlaced, or rather printed nested within each other, compared to separately 3D printing all components next to each other, a higher utilization rate can be achieved. This means that during 3D printing, a higher proportion which is melted and a lower proportion which stays as metal powder can be achieved. Thus, manufacturing time and costs can be reduced significantly.

Preferably, a plurality of adjustable intervertebral cages is 3D printed within one printing process. For this, the plurality of adjustable intervertebral cages is printed piece by piece next to each other. Thus, unit costs can be reduced.

Preferably, after step iv), the remaining unmolten metal powder on the substrate plate is removed therefrom.

The removal may be performed by suction the metal powder. Thus the remaining metal powder can be reused, which lowers the material costs.

Preferably, the removal is performed inside the chamber. Thus, it can be prevented that the remaining metal powder enters the machine hall, which can lower the maintenance costs.

Preferably, after step iv), the adjustable intervertebral cage is cut from the substrate plate by electrical discharge machining.

Thus, clear cutting edges can be achieved, which lowers the necessity for post-treatment. Additionally, the risk for damaging the cage, or rather cages can be decreased, because the load during removing the cages from the substrate plate is lowered.

If several cages are printed onto one substrate plate, electrical discharge machining can be used to simultaneously cut all cages placed on the substrate plate.

Preferably, basing points are printed onto the substrate plate additionally which are reference points for the cutting tool. Thus, high precision is enabled which can increase the component quality.

Preferably, a small support is left on the substrate plate, to prevent the cage from falling onto the EDM tool.

Preferably, after step iv), the adjustable intervertebral cage is cut by electrical discharge machining.

Thus, areas connecting single components of the cage can be cut to enable their separate movement. With electrical discharge machining a very precise cutting can be performed, which enables component tolerances. Thus, a high component quality can be achieved.

Additionally, with electrical discharge machining, cage geometries with angles above 45° can be realised. This can only be done for sections of the cage geometry which are accessible from the outside.

Further, EDM can be used to realise smooth surfaces, e.g. to enable low-friction sliding of two components against each other. Such smooth surfaces are distinguishably smoother than the untreated surfaces resulting from 3D printing.

If parallel, or rather vertical lifting of the moving plate is caused by a horizontal movement of the height wedge, a force perpendicular to the designated movement direction is acting between the moving plate and the base plate. This force leads to friction which can be lowered by smoothening the surface, e.g. by EDM cutting of a parallel sliding surface.

Preferably, the electrical discharge machining is performed with a wire. Thus, smooth surfaces can be achieved. The wire can be adjusted to the basing points.

Since with electrical discharge machining the metal is locally removed thermally, the width of the gap is bigger than the corresponding dimension of the cutting tool, e.g. the diameter of the wire. To cut a gap with a width of .25 mm, a wire with a diameter of .15 mm can be used.

Preferably, during steps iii) and iv), a rough structure is printed onto at least a portion of the base plate and/or the moving plate.

Preferably, the rough structure is on an outer, horizontal surface of the cage. Thus, grip between the adjacent vertebrae and the cage can be enhanced. Additionally, bone graft substitute fills the interspaces of the rough structure enhancing the effective area between the bone graft substitute and the cage. Thus, the fusing between the cage and the adjacent vertebrae is improved.

Preferably, the moving plate and the base plate comprise such rough structures. Thus, grip between the cage and the adjacent vertebrae, and fusing between the cage and the adjacent vertebrae can be improved at both horizontal sides of the cage. The rough surface can be manufactured directly by 3D printing.

Preferably, the rough structure is configured as a mesh, more preferably as a shallow mesh.

The rough surface can be a diamond structure (e.g., diamond 20-1.5), although other patterns may be used.

Preferably, during steps iii) and iv), the printed portion of the base plate, the moving plate, the height wedge and the lordosis wedge each are essentially spaced apart for at least 0.05 millimetres, preferably 0.08 millimetres, more preferably 0.1 millimetres and at most 5 millimetres, preferably 2.5 millimetres, more preferably 1 millimetres.

Thus, unwanted bonding between different components can be prevented while at the same time, the degree of remaining metal powder can be decreased. Accordingly, the component quality can be enhanced and at the same time the manufacturing costs can be lowered.

The problem underlying the present invention is also solved by a 3D printed adjustable intervertebral cage, comprising:
i) a moving plate having at least one first connector portion integrally formed in the moving plate;
ii) a base plate having at least one second connector portion integrally formed in the base plate;
iii) a height wedge having at least one third connector portion integrally formed in the height wedge, and the height wedge preferably having a moving section and a base section, wherein the height wedge is deformable so that the moving section is inclineable with regard to the base section; and
iv) a lordosis wedge, moveably supported with respect to the height wedge to control the inclination of the height wedge, the lordosis wedge is preferably arranged in contact with the moving section and the base section of the height wedge to control the inclination of the moving section,
wherein the connector portions of the moving plate, the height wedge and the base plate are interlaced within each other so as to maintain the structural stability of the adjustable intervertebral cage.

Structural stability means that the components of the cage do not fall apart under normal load.

Preferably, the height wedge is positioned proximal and the lordosis wedge is positioned distal. It is also possible to position the lordosis wedge proximal.

Preferably, the height wedge is configured to be one-pieced. Thus, manufacturing costs can be lowered.

To enable applying lordosis, preferably, the height wedge comprises a bearing section which is positioned between the moving section and the base section.

Preferably, the bearing section has a lower material thickness than its adjacent sections. Thus, the bearing section has a higher flexibility than its adjacent sections. Accordingly, the rotation axis is in the bearing section.

Preferably, the bearing section is configured as a living hinge. Thus, it can be easily manufactured by 3D printing.

Preferably, the cage comprises an insertion tip. Thus, gentle insertion is enabled resulting in less tissue damage. The insertion tip can be two-parted whereat one part is part of one plate, e.g. the moving plate and another part is part of the other plate, e.g. the base plate.

Preferably, the moving plate and the height wedge are coupled in such a way, e.g. by a wedge-in-wedge type connection, that inclination of the moving section results in an according inclination of the moving plate. Thus, inclination of the moving plate can be achieved. Accordingly, a lordosis can be set.

The wedge-in-wedge type connection can be realised by clicking elements or by a connection bolt.

The wedge-in-wedge type connection has the advantage that for enabling the functionality of the cage, less components have to be comprised therein which lowers the manufacturing costs. Additionally, a high level of precision and stability can be achieved with it.

Preferably, the cage is configured to distribute bone graft substitute.

Preferably, the exhaust ports are configured to evenly distribute the bone graft substitute around the cage. The more exhaust ports there are and the more even they are arranged on the surface of the cage, the more evenly the bone graft material can be distributed.

Preferably, the bone graft material is configured to enable stiff and strong fusing of the cage with its surrounding tissue, e.g. with the adjacent vertebral bodies. After approximately two to three months, the cage is fused with the surrounding tissue and the patient can begin to load its repaired spine again.

Preferably, all moving parts are supported one to another under pressure, thus the position of the height wedge and the lordosis wedge is fixed in every setting. Accordingly, under normal load, the cage is prevented from collapsing.

In most cases, during insertion and adjusting of the cage in a patient, first the parallel height is adjusted and afterwards the lordosis. Alternatively, first the lordosis can be set and afterwards the height.

Preferably, the height wedge and the lordosis wedge 400 are coupled in such a way that moving the height wedge automatically results in a similar movement of the lordosis wedge. Thus, the adjustment time can be shortened and the stability of the cage during its adjustment can be enhanced.

The adjustable intervertebral cage can have two long sides and two narrow sides perpendicular to the two long sides. Preferably, the inclination of the moving plate is set in such a way that one narrow side is lifted while the other narrow side remains at its initial height.

Preferably, the base plate comprises an interface to enable a connection between the cage and a corresponding instrument. The interface may be formed as a recess or a plurality of recesses. Thus, a safe connection between the instrument and the cage can be enabled. Additionally, the cage can be supported on the vertebrae with the base plate, which increases the stability during the insertion process of the cage.

Preferably, the lordosis wedge and the height wedge comprise latching recesses, wherein the adjustable intervertebral cage comprises latching noses corresponding to the latching recesses to prevent the adjustable intervertebral cage from collapsing.

It may be especially prevented from collapsing even if higher loads occur.

The latching nose or latching recess can be comprised within the height wedge or the lordosis wedge.

To enable the latching of the latching nose into the latching recess, the component comprising the latching nose is configured to be flexible in such a way that the latching nose is lifted if the component comprising the latching nose and the component comprising the latching recess are moved towards each other. Alternatively, it is also possible, that the component comprising the latching recess is configured to be flexible to enable the described functionality. The flexibility can be achieved by configuring the flexible component in such a way that it works as a leaf spring.

Preferably, the latching recess is configured to be beveled at one side in such a way that latching is supported. To prevent unwanted delatching, e.g. if a load is applied to the component comprising the latching nose and the component comprising the latching recess which would lead to moving them apart, the latching recess can be configured to be vertical at the other side. The same - one side beveled, the other one vertical - can be applied to the latching nose.

Preferably, the cage comprises multiple latching recesses arranged in a coherent row. Thus, multiple latching positions, or safety positions corresponding to multiple heights can exist.

The latching components can be positioned on the moving, or moved component, e.g. on the wedge. They can also be positioned on the component which supports the movement of the moving, or moved component, e.g. on the base plate. If a pair of corresponding latching components is provided, one latching component thereof is always comprised within a wedge. It is also possible that both corresponding latching components are comprised within wedges, e.g. a latching nose comprised within the height wedge and a latching recess comprised within the lordosis wedge.

Preferably, the adjustable intervertebral cage comprises at least one reset nose to unlock the latching recesses and latching noses at least temporarily.

Thus, controlled readjusting of the height wedge and/or the lordosis wedge is enabled. Accordingly, the insertion procedure is simplified.

Preferably, the first and second connector portions comprise a groove and/or a protrusion.

Preferably, the connector portions are positioned at opposing sides. Thus, tilting of the moving plate perpendicular to the lordosis can be prevented. If too many connector portions with the same direction of action are used, unwanted chocking of the moving plate with the height wedge can occur. Thus, preferably the height wedge and the moving plate comprise a maximum of four connector portions with the same action of direction each, whereof two of each are positioned at one side and the other two of each are positioned at the side opposite to the one side. Thus, both is prevented - unwanted detachment of the moving plate and unwanted chocking of the moving plate with the height wedge as well.

Preferably, the base plate and the moving plate comprise corresponding vertical connector portions. Thus, safe lifting of the moving plate can be additionally supported. The connector portions are preferably configured as vertical sliding means.

Preferably, the connector portion, or connector portions of the height wedge are positioned near the ramp, or ramps of the height wedge. Thus, the support of the parallel lifting of the moving plate finds place at the region where the load originates. Thus, distribution in the cage and eventual increase of the load can be prevented.

As an alternative to the vertical connector portion, the cage can comprise a connection spring which is configured to connect the moving plate to the base plate. The connection spring may be configured to enable vertical, or rather parallel lifting of the moving plate as well as its tilting to apply lordosis.

Preferably, the cage comprises two equivalent connection springs, whereof one is positioned at one side of the cage and the other one is positioned at the other side of the cage. Thus, tilting of the moving plate perpendicular to the lordosis can be prevented.

Thus, on the one side safe connection is enabled and on the other side, low resistance moving of the components relative to one another is enabled.

Preferably, one or more grooves comprise a printing recess which is formed deeper/wider than the rest of the groove.

Thus, the printing during manufacturing without metallically bonding the components is enabled. At the same time in the rest of the groove, a tighter fit is enabled, which results in a higher component quality.

Preferably, at least one of the first and second connector portions comprise an exhaust opening to outside of the moving plate and/or base plate.

Thus, metal powder remaining in the cage after 3D printing can be extracted more easily.

Preferably, the exhaust opening is positioned on a lateral surface of the moving plate. It is also possible to position the exhaust opening on a horizontal surface of the cage, preferably on the base plate or on the moving plate. Preferably, the cage comprises multiple exhaust openings. Thus, more areas inside the cage are reachable and the metal powder can be extracted more efficiently. It is also possible to configure an external connector portions, preferably a conical groove, in such a way that it can be additionally used as an exhaust opening. Therefore, the connector portion is deepened until a passage to the outside has been made.

Preferably, at least the moving plate and the height wedge comprise ramps which serve to adjust the height distance between the moving plate and the base plate.

Thus, the parallel height distance between the base plate and the moving plate is enabled. Accordingly, the cage can be precisely adjusted to the adjacent vertebrae.

The angle of the ramps in relation to the horizontal plane determines how much the moving plate is lifted by pulling it over a specific distance. The higher the angle, the more height is generated for a specific distance. Accordingly, the lower the angle, the more it has to be pulled to generate a specific height which results in longer wedges and therefore longer cages. Additionally, the higher the angle, the higher the resistance against pulling. Thus, an appropriate angle has to be set for which, the maximum length of the cage as well as the maximum resistance against pulling have to be considered. To lower the resistance against pulling, it is also possible to smoothen the surface of the ramps, e.g. by grinding. On the other hand, this would be an additional manufacturing step which increases the manufacturing costs.

Accordingly, the movements of the height wedge and the lordosis wedge include two adjustment ranges. One is for adjusting the parallel height, or the parallel distance between the two plates. The other is for adjusting the lordosis, or the distance between the two plates, relative to only one side of the plates at a time. Adjusting the lordosis causes the plates to tilt relative to each other. The tilt can be expressed as a lordosis angle.

Preferably, the height wedge and the lordosis wedge comprise locking receivers for receiving corresponding portions of an instrument for relatively moving the height wedge to the base plate and the moving plate, and relatively moving the lordosis wedge to the height wedge.

Preferably, the locking rod is configured to enable a bayonet lock between the locking section and the locking receiver. Thus, the locking rod is pushed into the cage to its locking position and rotated afterwards to lock with the locking receiver. The necessary angle with which the locking rod has to be rotated to lock the locking section with the locking receiver can be denoted as locking angle. Preferably, the surgeon has to rotate with a perceptible locking angle. Thus, unwanted locking can be prevented. Such locking angle is preferably higher than 15°, e.g. approximately 90°.

Thus, the adjusting of the cage can be performed outside of the spine, which enables a more precise adjusting.

The problem underlying the present invention is also solved by an alternative 3D printed adjustable intervertebral cage, comprising
a first moving plate having first adjustment means and second adjustment means and a second moving plate having first adjustment means and second adjustment means ;
a height wedge for adjusting the distance between the first and second moving plates, having first and second interacting means interacting with the first adjustment means of the first and second moving plate, respectively; and
a lordosis wedge for adjusting the inclination of the first and second moving plate, the lordosis wedge comprises first and second interacting means interacting with the second adjustment means of the first and second moving plate, respectively.

Thus, the lordosis and height adjustable by a specific translational movement of the lordosis wedge and the height wedge can be doubled as compared to a cage with just a single moving plate.

With the use of two moving plates, the range of motion can be enhanced. Such cage can be used for applications where range of motion has a greater significance than the stability during the surgical procedure.

Preferably, the first adjustment means of the first and second moving plate and the first and second interacting means of the height wedge are formed as grooves and projections and/or as ramps.

Thus, safe adjusting of the cage is enabled.

Preferably, the grooves are formed on both inner sides of the moving plates and the projections are formed on both outer sides of the height wedge.

Thus, safe adjusting of the cage is enabled.

Preferably, the second adjustment means of the moveable plate and the first and second interacting means of the lordosis wedge are formed as ramps.

Thus, safe adjusting of the cage is enabled.

Preferably, the height wedge and/or the lordosis wedge comprise a bore for introducing an instrument.

Thus, safe adjusting of the cage is enabled.

Preferably, the first traction bolt is arranged within the lordosis wedge and the height wedge to fixedly connect them and a second traction bolt is arranged within the height wedge and the moving plates to fixedly connect the height wedge with the moving plates.

Thus, safe adjusting of the cage is enabled.

The problem underlying the present invention is also solved by an alternative 3D printed adjustable intervertebral cage, comprising
a moving plate having first adjustment means;
a base plate rotatably connected with at least the moving plate, preferably on a rotation axis parallel to and on the long side of the adjustable intervertebral cage;
a height wedge for adjusting the distance between the moving plate and the base plate, the height wedge comprises a moving section and a base section which are rotatably connected to each other, preferably on the same rotation axis as the rotation axis of the moving plate and the base plate, wherein the height wedge further comprises interacting means for interacting with the adjustment means of the moving plate to adjust said distance and second adjustment means; and
a lordosis wedge for adjusting the inclination of the moving plate, the lordosis wedge is moveable relative to the height wedge and comprises second interacting means interacting with the second adjustment means of the height wedge, to adjust the inclination of the moving plate by rotating the moving section of the height wedge.

Thus, adjusting the lordosis on the long side of the adjustable intervertebral cage is enabled.

Preferably, the adjustment means and the interacting means are formed as ramps.

Thus, safe adjusting of the cage is enabled.

Preferably, the adjustable intervertebral cage further comprising latching screws, wherein a first latching screw fixes the base section of the height wedge to the base plate in at least one translational direction and a second latching screw fixes the base section of the height wedge to the lordosis wedge in at least one translational direction.

Thus, safe adjusting of the cage is enabled.

Preferably, the latching screws comprise a head with hook elements and a pin with hook elements that interact with corresponding hook elements on the base plate, the lordosis wedge and the height wedge.

Thus, safe adjusting of the cage is enabled.

Preferably, the lordosis wedge and the height wedge comprise accommodation portions for interaction with an instrument.

Thus, safe adjusting of the cage is enabled.

The problem underlying the present invention is also solved by an instrument for adjusting an adjustable intervertebral cage with a height wedge and a lordosis wedge comprising:
i) a fixation device shiftable into a fixed position and configured to be fixable with the adjustable intervertebral cage;
ii) a first locking rod configured to interlock with the height wedge and/or the lordosis wedge, and configured to transmit its movement into a movement of the height wedge and/or the lordosis wedge;
iii) a fixation handle configured to shift the fixation device into the fixed position; and
iv) an adjustment handle comprising a first adjustment mean, wherein the first adjustment mean is configured to move the first locking rod.

Thus, the adjustable intervertebral cage can be precisely and safely adjusted with the corresponding instrument.

If a wedge is locked to its corresponding locking rod, moving the locking rod results in a movement of the wedge. The initial movement of the locking rod can be rotational or translational. A translational movement can be transmitted directly to the wedge which is then pulled toward or pushed away from the surgeon. A rotational movement of the instrument, or rod, can be transformed into a translational movement of the wedge via a thread.

Preferably, the locking rod comprises a locking section. Preferably, the locking section is bulging, e.g. shaped like a hammer head.

Preferably, the instrument comprises a second locking rod and the adjustment handle comprises a second adjustment mean configured to move the second locking rod, wherein the first locking rod is configured to interlock with the height wedge, and configured to transmit its movement into a movement of the height wedge, and wherein the second locking rod is configured to interlock with the lordosis wedge, and configured to transmit its movement into a movement of the lordosis wedge.

Thus, separate adjusting of the lordosis wedge and the height wedge is enabled. Accordingly, a more precise adjusting of the cage dimensions to the corresponding adjacent vertebrae can be performed.

Preferably, the locking rods are coupled in such a way that they can only be rotated together. Thus, if one locking rod is positioned inside another locking rod and the locking rods are rotated into locking position, misuse which e.g. can lead to damaging the cage, can be prevented.

Preferably, the instrument comprises a rotation mean, with which the locking rod, or rather locking rods can be rotated.

Preferably, the instrument comprises a fixation slot, with which the rotation mean can be fixed in a specific position, e.g. in the locking position or in the insertion position. The rotation mean can be configured as a rotation pin.

If the instrument comprises two locking rods positioned side by side, the instrument comprises two rotation means, each connected to one locking rod. The two locking rods are fixed in the same fixation slot. To enable separate movement of the two rotation means, one can be configured to be rotatable into one direction (e.g. clockwise) while the other can be configured to be rotatable in the opposite direction (e.g. counterclockwise).

Preferably, the adjustment handle comprises a first transmission and optionally a second transmission, wherein the first transmission is connected to the first adjustment mean and the first locking rod, and wherein optionally the second transmission is connected to the second adjustment mean and the second locking rod.

Preferably, the first and optionally the second transmission transmit a rotational movement of the corresponding adjustment mean into a translational movement of the corresponding locking rod. Thus, pulling or pushing the cage away from its insertion position can be prevented. Accordingly, a safer insertion procedure of the cage is enabled.

Preferably, the adjustment mean can be rotated clockwise and counterclockwise which leads to translational movement of the locking rod into one direction (clockwise) and in the opposite direction (counterclockwise). Thus, readjusting of the height, or rather the lordosis is enabled.

Preferably, the adjustment mean comprises a manipulation portion configured to manipulate the adjustment mean, e.g. to rotate it.

Preferably, the instrument is configured to get reversibly disassembled into its individual parts. Thus, cleaning of the instrument can be simplified.

Preferably, such transmission is a combination of two threads, e.g. an internal thread and an external thread. Preferably, the internal thread is comprised within a structural component of the adjustment handle, while the external thread is comprised within an adjustment handle.

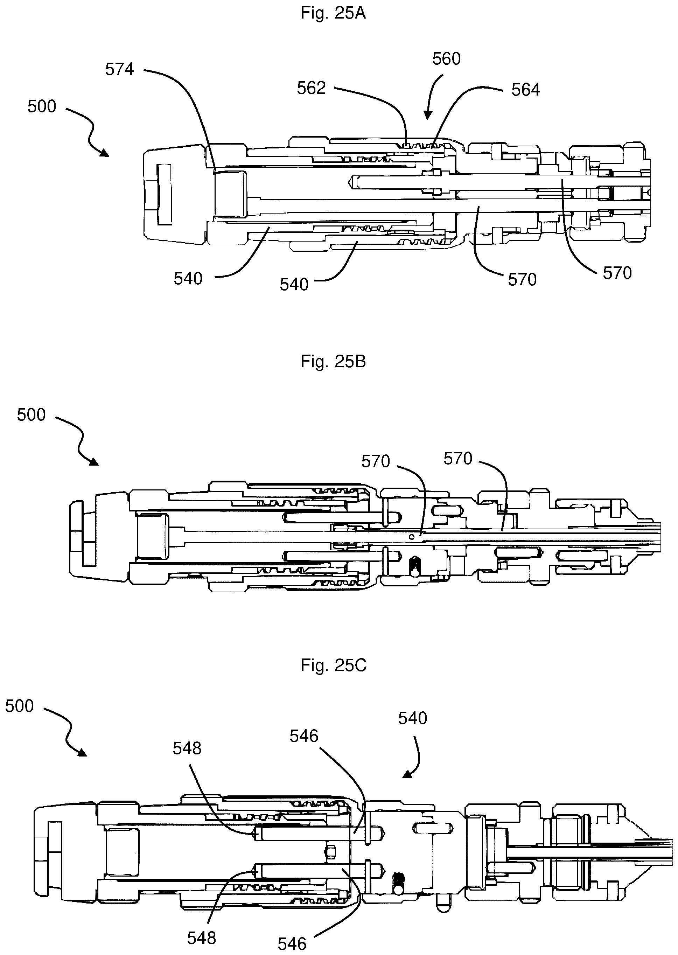

Preferably, the adjustment handle further comprises a guiding rod configured to guide the translational movement of an adjustment mean, or the adjustment means (fig. 25C). Thus, unwanted chocking of the locking rods within the shaft can be prevented. Preferably, the adjustment handle comprises multiple guiding rods, e.g. two guiding rods. Preferably, the adjustment handle comprises a recess, or recesses, configured to take up the guiding rod, or guiding rods. Thus, very precise guiding can be enabled.

Preferably, the thread height is adapted to fit the necessary sensitivity of the translational adjustability of the locking rods. The more sensitively the locking rod has to be adjustable in a translational direction, the lower the thread height should be set, e.g. a fine thread or a precision thread can be used.

Preferably, the connection between the first and/or second locking rod and the first and/or second adjustment mean is a plug connection where the plugged-in locking rod is fixed by a clicking mechanism and/or a threading mechanism. Preferably, the threading mechanism corresponds to the threads relating to the first and/or second transmission. Thus, the necessary number of components of the instrument can be lowered, which lowers its manufacturing costs.

Preferably, the components of the instruments are manufactured by machining, e.g. by turning or milling.

Preferably, the first adjustment handle comprises a first position indicator configured to indicate the position of the first locking rod, wherein optionally the second adjustment handle comprises a second position indicator configured to indicate the position of the second locking rod.

Preferably, the adjustment handle comprises an indication recess configured to reveal the indication portion.

Preferably, the indication portion and the indication recess form a position display.

Preferably, the instrument comprises a shaft configured to cover the first locking rod and/or the second locking rod. Thus, the first and/or optionally the second locking rod can be protected from external disturbances. Accordingly, a safer adjusting of the adjustable intervertebral cage can be achieved.

### Brief description of the figures

Fig. 1 shows an adjustable intervertebral cage in an isometric cross sectional view.
Fig. 2 shows a height wedge with four ramps and a bearing section.
Fig. 3 shows a moving plate with four ramps.
Fig. 4A shows an adjustable intervertebral cage without a moving plate in an isometric view.
Fig. 4B shows a moving plate with two latching noses from its lower side.
Fig. 4C shows a moving plate with two latching noses in an isometric cross sectional view.
Fig. 5A shows an adjustable intervertebral cage without a moving plate with two locking receivers.
Fig. 5B shows an adjustable intervertebral cage in an isometric cross sectional view with a corresponding pair of locking rods outside of the adjustable intervertebral cage.
Fig. 5C shows the configuration of fig. 5B, whereat the pair of locking rods is inside the adjustable intervertebral cage.
Fig. 6 shows an instrument 500.
Fig. 7 shows an adjustable intervertebral cage with three reset noses.
Fig. 8A shows a moving plate with two conical grooves and a vertical sliding mean in an isometric view from below.
Fig. 8B shows an adjustable intervertebral cage without a moving plate and a lordosis wedge corresponding to the moving plate shown in fig. 8A with two conical pins and a vertical sliding mean.
Fig. 9 shows an adjustable intervertebral cage with four exhaust openings and an insertion tip in an isometric view.
Fig. 10 shows a fixation device in an isometric view.
Fig. 11 shows an instrument without an adjustment handle.
Fig. 12A shows an alternative adjustable intervertebral cage with five parallel sliding surfaces in an isometric view.
Fig. 12B shows the adjustable intervertebral cage displayed in fig. 12A in a top view.
Fig. 13A shows the adjustable intervertebral cage displayed in fig. 12A with two latching screws in a cross sectional view.
Fig. 13B shows one of the latching screws displayed in fig. 13A in an isometric view.
Fig. 14A shows an adjustment mean in a frontal view.
Fig. 14B shows an adjustment handle with an adjustment mean and a position display in a frontal view.
Fig. 15A shows a lordosis wedge with three sliding means in an isometric view.
Fig. 15B shows a height wedge with two sliding means in an isometric view.
Fig. 15C shows a base plate with two horizontal sliding means in an isometric view.
Fig. 16 shows a height wedge with three runners in an isometric view from below.
Fig. 17 shows the adjustable intervertebral cage displayed in fig. 12A in an isometric cross sectional view with a joint pin and two slotted holes.
Fig. 18 shows the height wedge displayed in fig. 16 with two cutting slots in an isometric view.
Fig. 19A shows the front part of an instrument corresponding to the adjustable intervertebral cage displayed in fig. 12A with two adjacent locking rods in a frontal view.
Fig. 19B shows the front part of an instrument corresponding to the adjustable intervertebral cage displayed in fig. 1 with two locking rods positioned within each other in a frontal view.
Fig. 20 shows an instrument corresponding to the adjustable intervertebral cage displayed in fig. 12A in an isometric view.
Fig. 21A shows a lordosis wedge corresponding to an alternative adjustable intervertebral cage in a frontal view.
Fig. 21B shows a first moving plate corresponding to an alternative adjustable intervertebral cage in an isometric view.
Fig. 21C shows a second moving plate corresponding to an alternative adjustable intervertebral cage in an isometric view and corresponding to the first moving plate displayed in fig. 21B.
Fig. 22 shows an alternative adjustable intervertebral cage corresponding to the components displayed in fig. 21A-C in an isometric view.
Fig. 23 shows the adjustable intervertebral cage displayed in fig. 22 with three twist locks and a rough structure in an isometric view.
Fig. 24A shows the adjustable intervertebral cage displayed in fig. 22 with two traction bolts in a cross sectional view.
Fig. 24B shows the height wedge corresponding to the adjustable intervertebral cage displayed in fig. 22 with two threads in an isometric cross sectional view.
Fig. 24C shows the height wedge corresponding to the adjustable intervertebral cage displayed in fig. 22 with four ramps in an isometric view.
Fig. 25A shows the rear part of an instrument corresponding to the adjustable intervertebral cage displayed in fig. 12A in a cross sectional view.
Fig. 25B shows the rear part of an instrument corresponding to the adjustable intervertebral cage displayed in fig. 1 in a cross sectional view.
Fig. 25C shows the rear part of the instrument displayed in fig. 25B in another cross sectional view.
Fig. 26A shows a 3D printer in a schematic view.
Fig. 26B shows an EDM configuration in a schematic view.

### Description of the preferred embodiments

The cages are manufactured by Selective Laser Melting (SLM), e.g. with a 3D printer 100, as shown in figure 25A. Therefore, a substrate plate 110 is fastened to an indexing table 120 inside a chamber 130 with a controlled atmosphere of inert gas (e.g. argon or nitrogen). Metal powder 105 is applied flat to the substrate plate 110 as a layer. The metal powder 105 is preferably a titanium alloy, e.g. Ti-6AI-4V to enable biocompatibility. Each 2D slice of the cage is fused by selectively melting the metal powder 105 via a laser. The laser has enough energy to fully melt or rather weld the metal particles to form solid metal. The substrate plate 110 is lowered by the layer thickness (z-direction). New metal powder 105 is applied and the process is repeated layer by layer until the part is complete. The completed part is removed from the substrate plate 110 by cutting or breaking off.

Since the metal powder 105 is locally melted, the right amount of heat dissipation is important. If too less heat is dissipated before applying a new layer, exact geometry curves cannot be realized since newly applied metal powder 105 is melted by the lower layer. If too much heat is dissipated before applying a new layer, the bond between the layers will be too weak, resulting in low component strength.

Since a new layer has to be connected to its lower layer, overhangs cannot be realized by SLM. The highest angle possible between 0° (parallel to the z-direction and directed towards the top of the cage) and 90° (perpendicular to the z-direction) is 45°. If angles above 45° are necessary, supports have to be used.

All components of the cage are printed interlaced, or rather nested within each other.

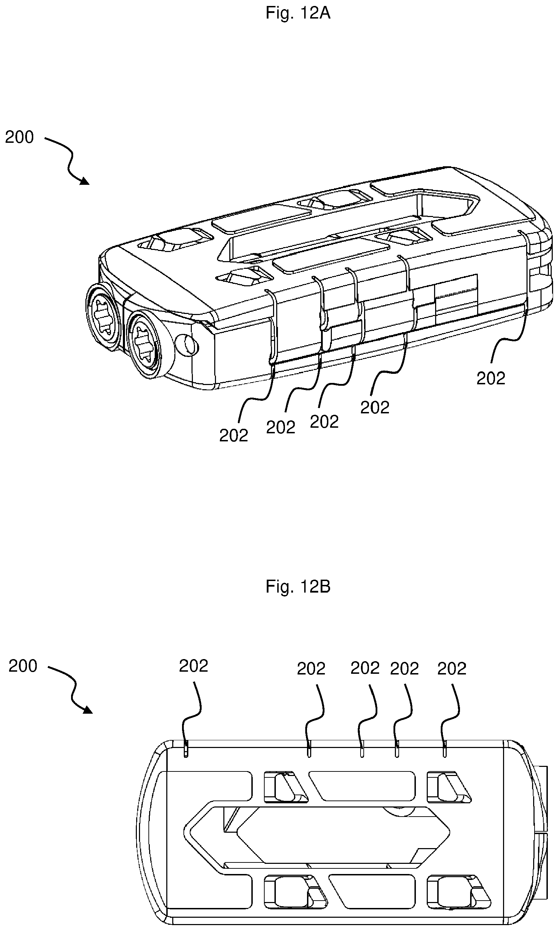

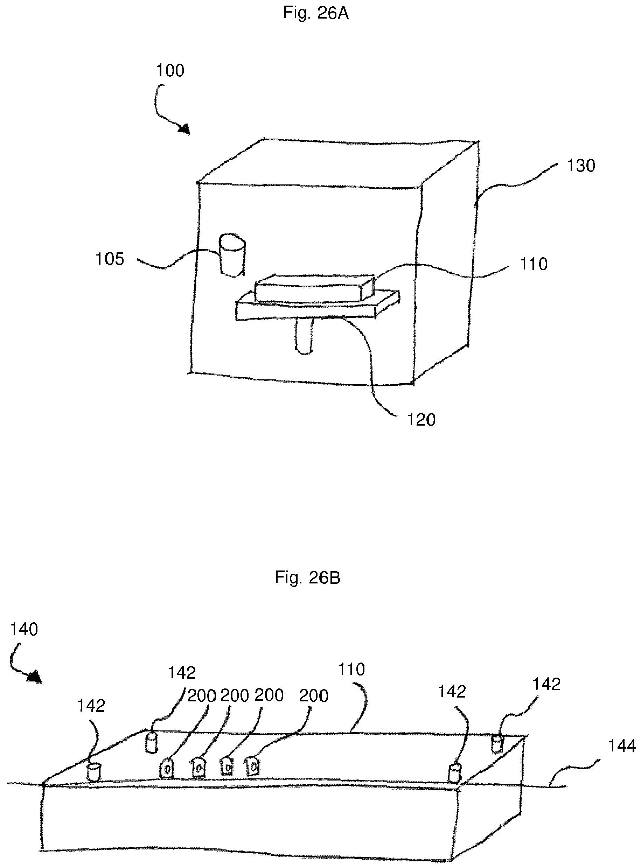

After 3D printing, areas connecting single components of the cage are cut by electrical discharge machining (EDM) to enable their separate movement (figs. 12A, 12B). Such EDM configuration 140 is shown in fig. 26B.

With EDM, the cage is also removed from the substrate plate 110.

To enable high precision for cutting the cages from the substrate plate 110, several basing points 142, e.g. four basing points 142, are additionally printed onto the substrate plate 110. As an EDM tool, a wire 144 can be used. This wire 144 can be adjusted to the basing points 142.

The components of the instruments 500 are manufactured by machining, e.g. by turning or milling.

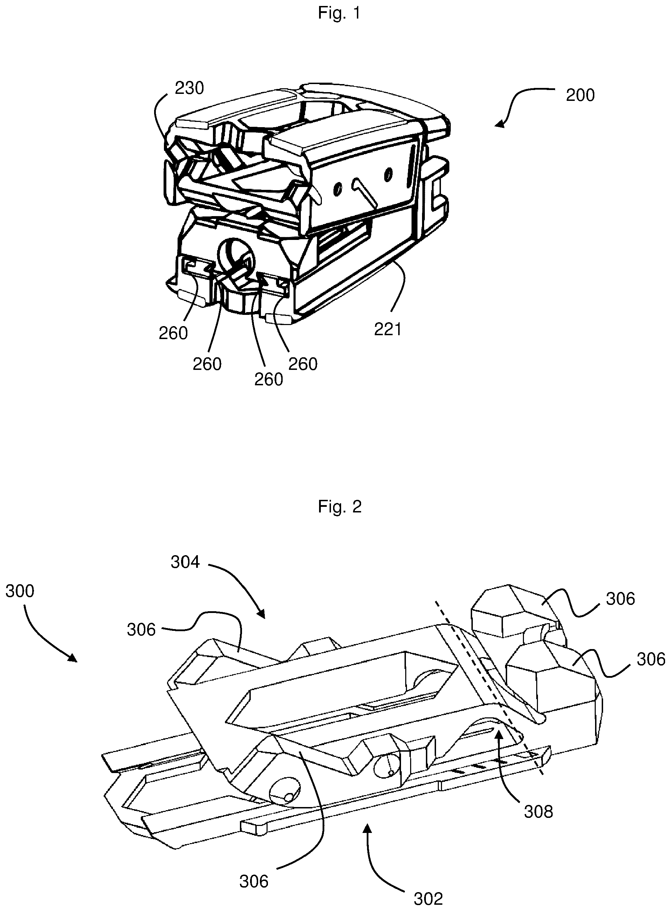

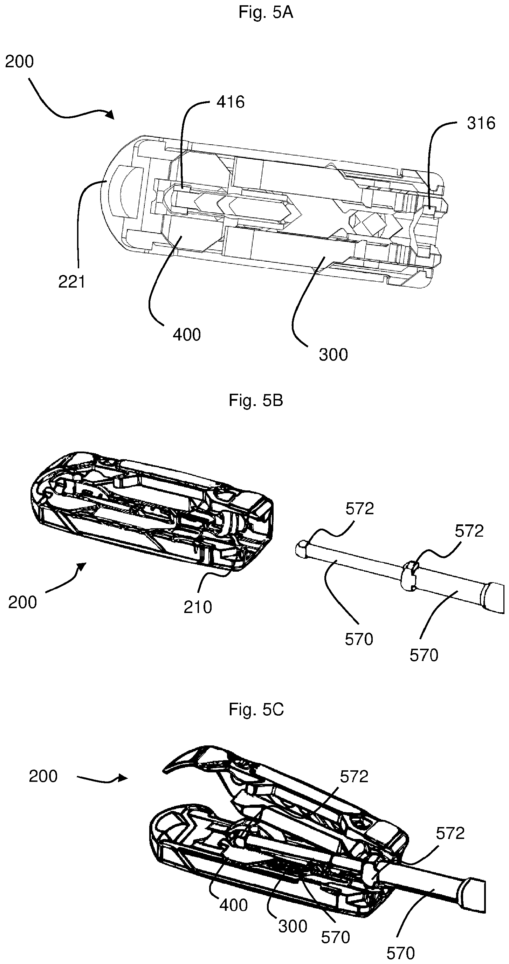

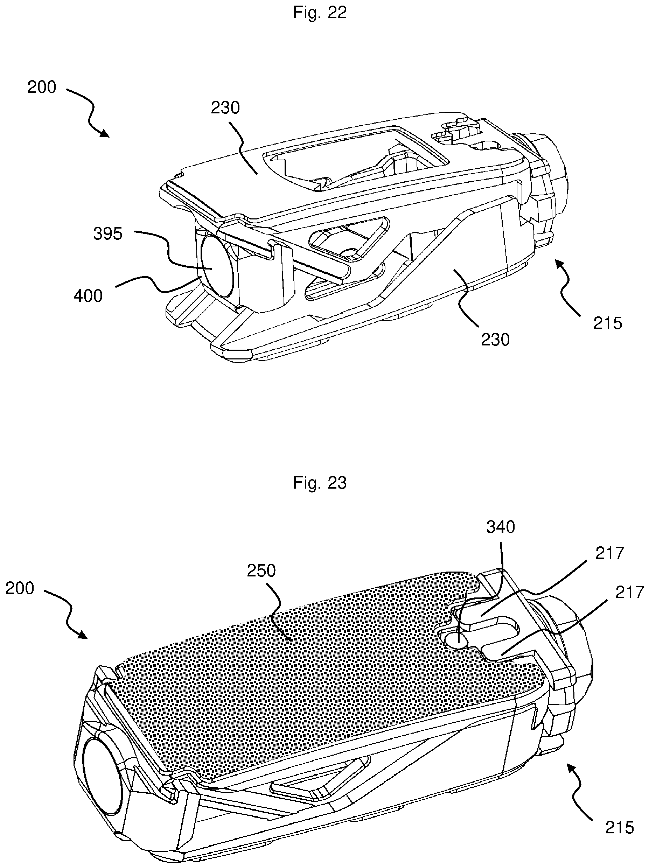

The adjustable intervertebral cage 200 comprises a base 215 (fig. 22) which can be formed as a base plate 221 and at least one moving plate 230 (fig. 1). The base 215 (fig. 22) comprises an interface 210 to enable a connection between the cage and an instrument 500 (fig. 5B). The interface 210 is formed as a recess or a plurality of recesses.

The outer structure of the cage is formed by a moving plate 230 and a base plate (fig. 1) or two moving plates (fig. 22).

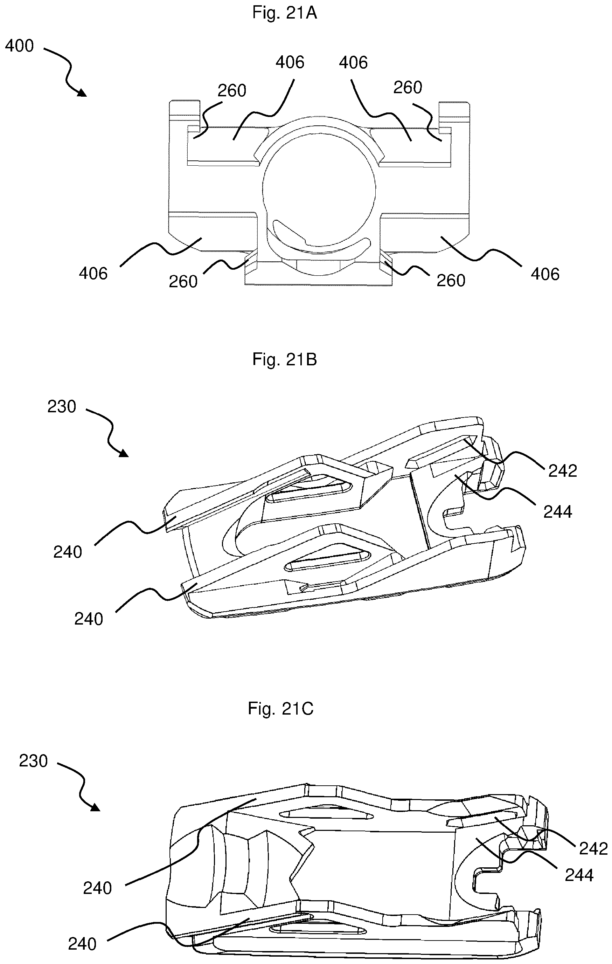

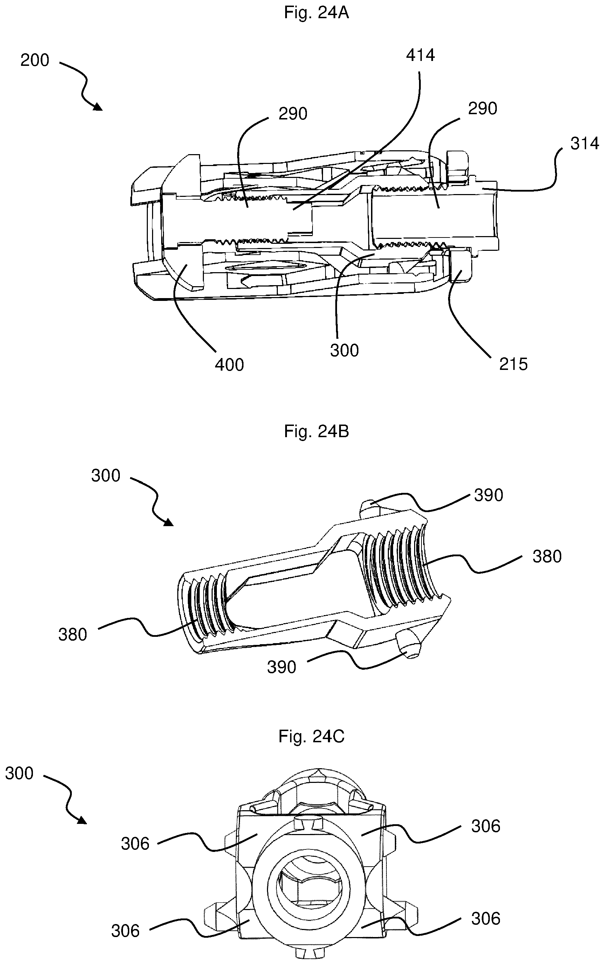

If two moving plates 230 are comprised within the cage, the height wedge 300 and the lordosis wedge 400 each comprise four ramps 306, 406 on their upper (two ramps) and their lower (two ramps) side (figs. 21A, 24C).

An adjustable intervertebral cage 200 according to the present invention further comprises a height wedge 300 and a lordosis wedge 400 disposed between and abutting the plates. The height wedge 300 and the lordosis wedge 400 is moveable in the adjustable intervertebral cage 200 by the instrument 500. To enable such movement, the cage can comprise several guiding rails 260. The guiding rails 260 can be part of a plate or another wedge. To enable the connection between the instrument 500 and the height wedge 300 and lordosis wedge 400, the height wedge 300 and the lordosis wedge 400 each comprise a locking receiver 316, 416 (fig. 5A).

The height wedge 300 is positioned proximal and the lordosis wedge 400 is positioned distal (fig. 1).

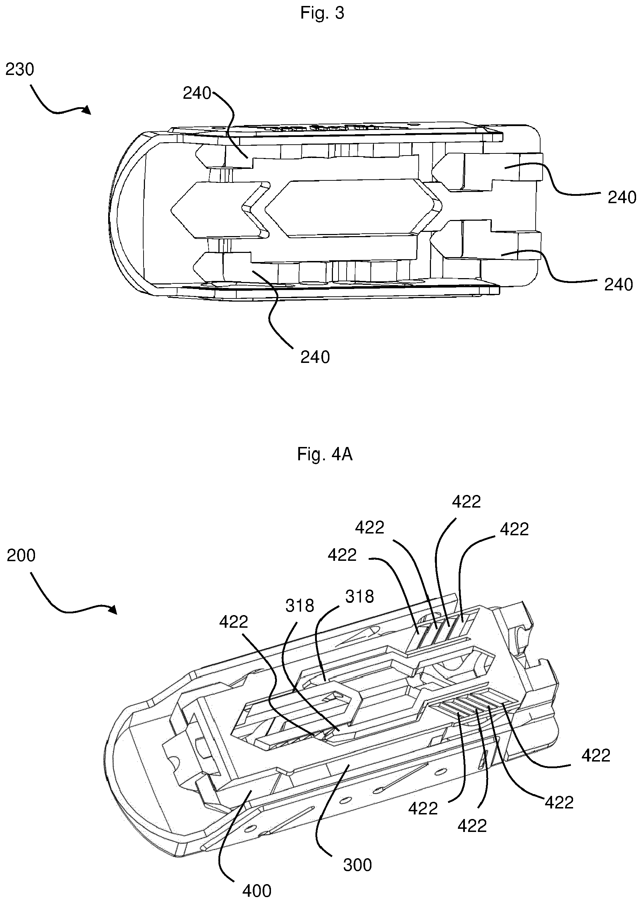

The height wedge 300 comprises four ramps 306 (fig. 2). The moving plate 230 comprises as many ramps 240 as the height wedge 300, namely four ramps 240. The ramps 240 of the moving plate 230 are shaped to fit the corresponding ramps 306 of the height wedge 300 (fig. 3).

The cage 200 comprises a parallel sliding surface 202 to enable moving its single components. The parallel sliding surface 202 is manufactured by EDM to provide a smooth surface. With the parallel sliding surface 202, parallel lifting of the moving plate 230 is supported.

The cage comprises five parallel sliding surfaces 202 (figs. 12A, 12B).

The lordosis wedge 400 comprises four ramps 406 to enable one-sided lifting of the moving plate 230 to increase lordosis of the cage (figs. 21A-C). These ramps 406 are supported on the moving plate 230. Alternatively, the lordosis wedge comprises two ramps 406, which are supported on the height wedge 300 (fig. 2) and thus indirectly on the moving plate 230 as well.

In fig. 24A, the coupling between lordosis wedge 400 and height wedge 300 is direct.

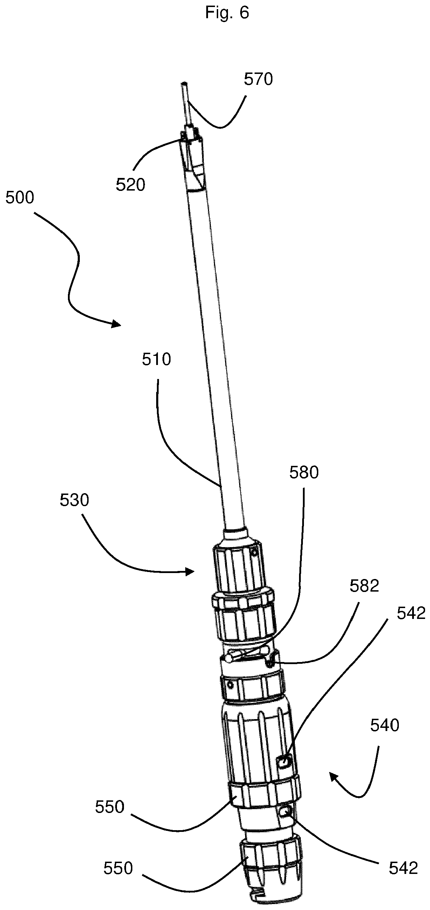

An alternative is displayed in figs. 6, 24A, 24B, wherein indirect coupling is realised by interconnecting the locking rods 570 in the adjustment handle 540. Therein, the coupling is indirect by coupling the locking rods 570 in such a way that pulling the locking rod 570 corresponding to the locking wedge automatically results in a similar movement of the locking rod 570 corresponding to the lordosis wedge 400. The lordosis wedge 400 itself can be moved actively and independently from the height wedge 300. Thus, it is enabled to adjust the lordosis, or the distance between the two plates, relative to only the side of the plates at a time, where the lordosis wedge 400 is positioned.

In fig. 24A, direct coupling of the height wedge 300 and the lordosis wedge 400 is enabled by screwing the height wedge 300 and the lordosis wedge 400 together. For this configuration, the cage comprises two traction bolts 290, one of it configured to pull the lordosis wedge 400 and the other one configured to pull the height wedge 300. The traction bolts 290 are configured to pull the height wedge 300 and the lordosis wedge 400 towards the instrument 500 by screwing it into a thread 380. The threads 380 are comprised within the height wedge 300 (Fig. 24B). The head of the traction bolt 290 for pulling the lordosis wedge 400 is supported on the lordosis wedge 400. The head of the traction bolt 290 for pulling the height wedge 300 is supported on the base 215. To manufacture such cage, the traction bolts 290 are 3D printed separately to enable the necessary threadability. The threads 380 are cut into the height wedge 300, after 3D printing.

The traction bolt 290 comprises a mating feature 314, 414 - her displayed as a hexalobe - for mating with the instrument 500. Thus, the traction bolt 290 can be screwed into the height wedge 300, or lordosis wedge 400 (fig. 24A).

If a rotational movement of the instrument 500 is transformed into a translational movement of the wedge without a locking rod 570, the cage 200 comprises a twist lock 217, 340 to prevent twisting of the entire cage during the surgical procedure (fig. 23). The cage comprises multiple twist locks 217, 340 acting in every rotational direction (clockwise and counterclockwise). The twist locks 217, 340 are positioned at two opposite sites around the rotational axis. Accordingly, the height wedge 300 comprises two twist locks 340 - one at each side - configured as pins while the base 215 comprises four twist locks 217 - two at each side - configured as tongues. Accordingly, the cage does not comprise a locking receiver 316, 416.

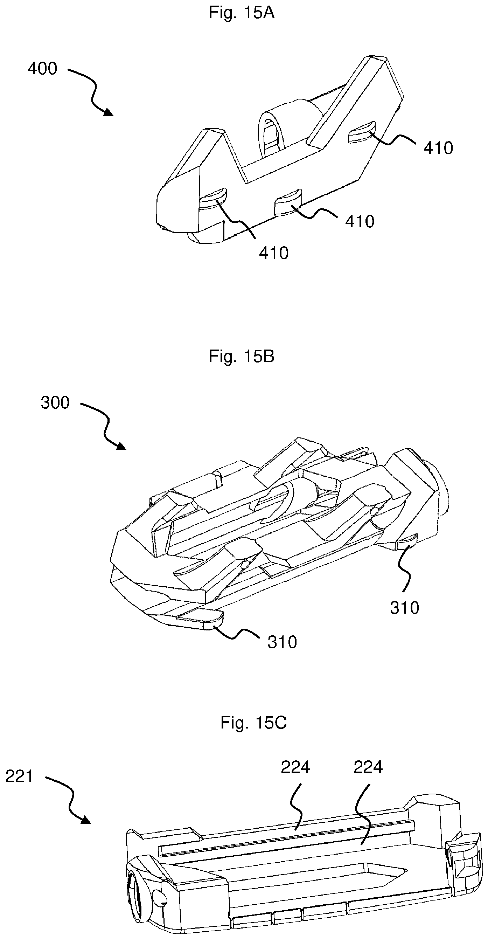

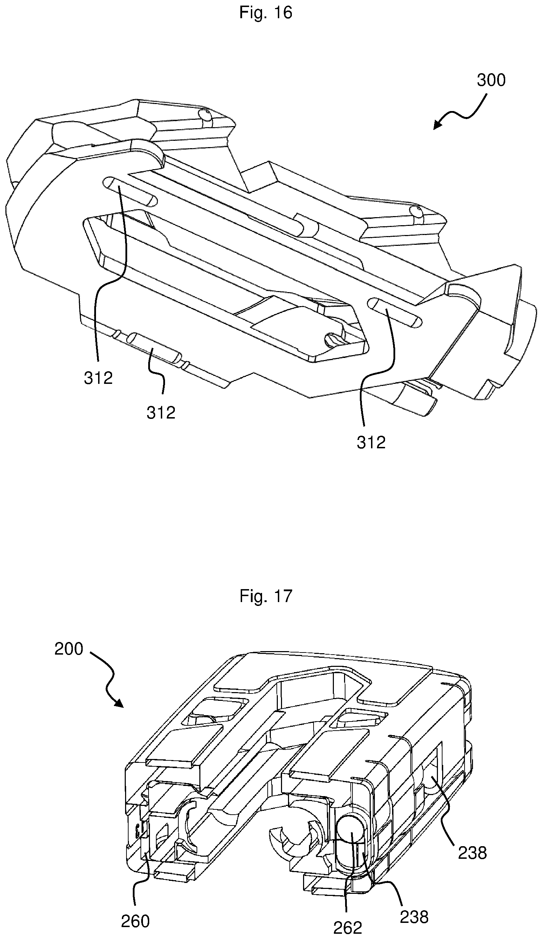

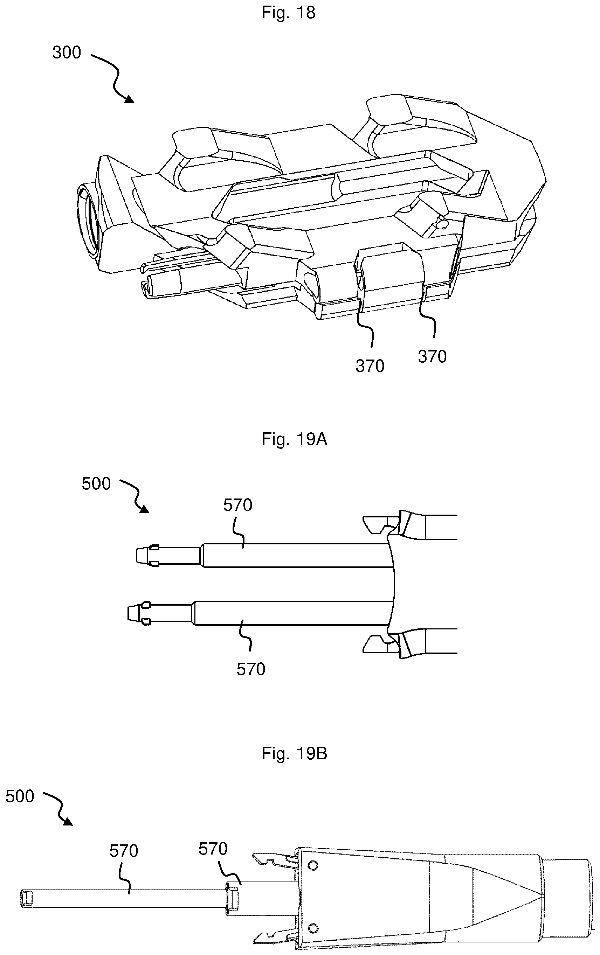

The movement of the height wedge 300 and the lordosis wedge 400 is guided by the guiding rails 260 (fig. 1). Each movement is guided by two guiding rails 260 (figs. 1, 15) or by four guiding rails 260 (fig. 21A). The guiding rail 260 is formed as a joint pin 262, which additionally can be used to enable the rotation of the moving plate 230 attached thereto (fig. 17). To enable both movements - rotation and lifting - of the moving plate 230 attached to the bottom plate by the joint pin 262, the moving plate 230 comprises a slotted hole 238, wherein the joint pin 262 is positioned. The guiding rails 260 are comprised within the base plate 221 or within two moving plates 230. The guiding rails 260 can also be comprised within the height wedge 300 (fig. 1). With such guiding rails 260, only the lordosis wedge 400 can be guided accordingly. If a joint pin 262 is used for enabling a rotation of the moving plate 230 attached thereto, the height wedge 300 is two-parted while its two parts are connected via the joint pin 262. To enable separate movement of the two parts of the height wedge 300 to set lordosis, after 3D printing, the height wedge 300 is cut by EDM, resulting in cutting slots 370 comprised within the height wedge 300 (fig. 18).

If a guiding rail 260 is comprised within the base plate 221 and configured to guide the horizontal movement of the height wedge 300, the guiding rail 260 additionally prevents the height wedge 300 from unwanted tilting away from the base plate 221, especially if lordosis is adjusted (fig. 17).

The height wedge 300 further comprises runners 312 positioned at the surface of the height wedge 300 adjacent to the horizontal surface of the base plate 221 and configured to lower the friction between the height wedge 300 and the base plate 221 (fig. 16). Alternatively, the runners 312 can also be comprised within the base plate 221.

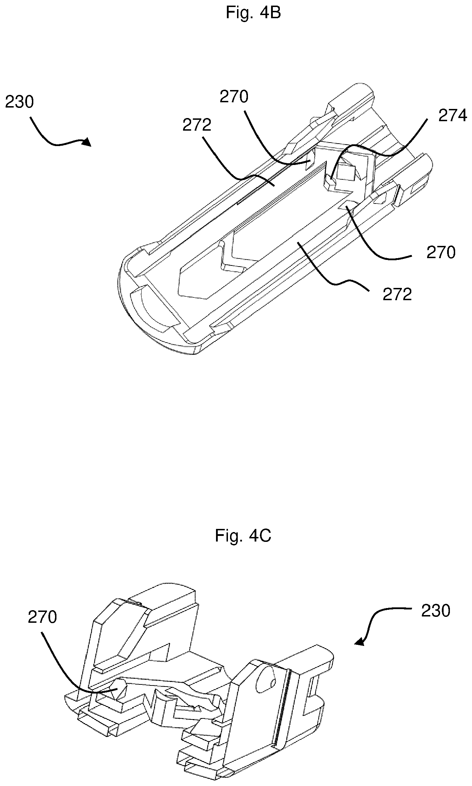

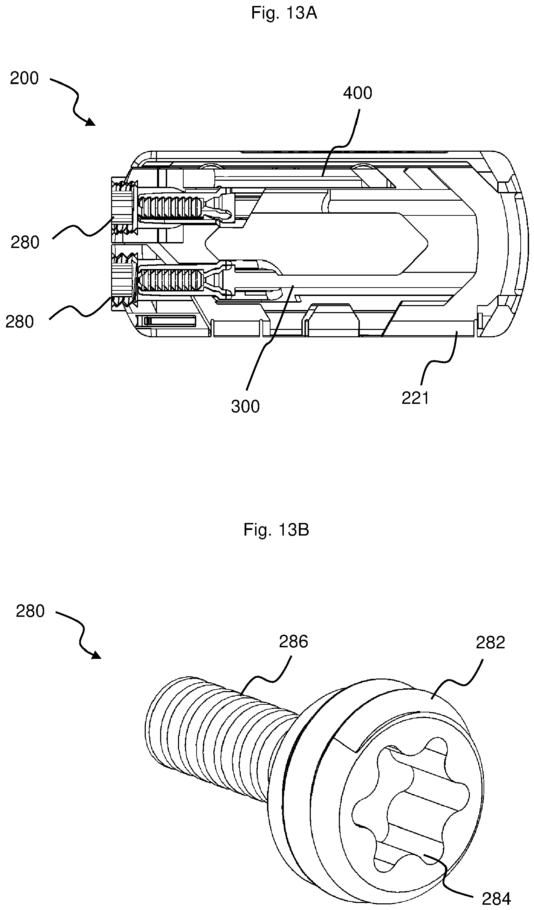

The cage further comprises a pair of a latching nose 270, 318 and a latching recess 422 corresponding to the latching nose 270, 318 (figs. 4A, 4B). The latching component can be comprised within a wedge (fig. 4A). The latching component can also be a separate component (figs. 13A, 13B). This separate latching component is a latching screw 280. The latching screw 280 comprises a threaded part 282 and a latching part 286 (fig. 13B). Preferably, the threaded part 282 comprises a fastener receptacle 284 configured to fix the latching screw 280 to the cage by screwing in. If the latching part 286 of the latching screw 280 is interacting with a latching nose of a cage 270, 318, before screwing in the latching bolt, the latching nose 270, 318 has to be put into reset position (fig 13A).

In fig. 4A, the height wedge 300 comprises two equivalent latching noses 318 which are positioned opposite each other.

In fig. 4B, two leaf springs 272 are displayed which enable flexibility of the latching noses 270.

In fig. 4A, the latching recesses 422 are configured to be beveled at one side in such a way that latching is supported. To prevent unwanted delatching, e.g. if a load is applied to the component comprising the latching nose 270, 318 and the component comprising the latching recess 422 which would lead to moving them apart, the latching recess 422 is configured to be vertical at the other side. The same - one side beveled, the other one vertical - is be applied to the latching nose 270, 318.

In fig. 4A, it can be seen that the cage comprises multiple latching recesses 422 arranged in a coherent row. Thus, multiple latching positions, or safety positions corresponding to multiple heights exist.

As displayed in fig. 4A, both corresponding latching components are comprised within wedges. Thus, the latching noses 318 are comprised within the height wedge 300 and the latching recesses 422 are comprised within the lordosis wedge 400.

In fig. 4B it can be seen, that the cage further comprise a reset nose 274, which is configured to release the latched latching components in a controlled manner if it is pushed. Thus, the height and the lordosis can be readjusted if they are adjusted above their specific target value. Pushing the reset nose 274 results in pushing the latching noses 270 apart from the latching position. Thus, the component comprising the latching component can be readjusted. The reset nose 274 is positioned near and attached to the leaf springs 272.

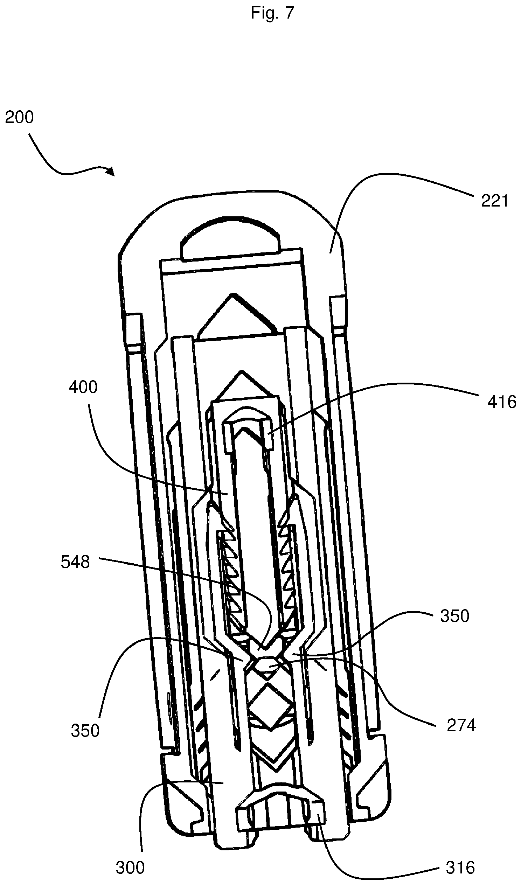

In fig. 7, three reset noses 274, 350 can be seen. If the two reset noses 350 attached to the height wedge 300 are pushed, the lordosis wedge 400 can be readjusted. They can be pushed by rotating the locking rod 570 corresponding to the lordosis wedge 400 before it has reached the locking position, e.g. before entering the cage, and then push the locking rod 570 into the cage until it has reached the reset noses 350. If the reset nose 274 attached to the base plate 221 is pushed, the height wedge 300 can be readjusted. It can be pushed by pushing the corresponding locking rod 570 into the cage beyond the locking position without rotating it. Here it can be seen, that the reset nose 274 for readjusting the height wedge 300 is positioned on the base plate 221 and not on the moving plate 230 because the moving plate 230 is lifted from the base plate 221. This would result in lifting the corresponding reset nose 274 away from the locking rod 570 which then cannot reach and push the reset nose 274. The lordosis wedge 400 comprises a recess 420 to enable that the corresponding locking rod 570 can reach the reset position, especially if the lordosis wedge 400 is pulled to the farthest position inside the green wedge.

In fig. 2, a one-pieced height wedge 300 is displayed, which comprises a base section 302 and a moving section 304. To enable one-sided lifting of the moving plate 230, the moving section 304 of the height wedge 300 is rotated around a rotation axis 309. Thus, the region of the height wedge 300 which is distant to the rotation axis 309 is lifted and the region which is near the rotation axis 309 stays more or less in its initial state.

In fig. 2 it can be seen, that the height wedge 300 comprises a bearing section 308 which is positioned between the moving section 304 and the base section 302. The bearing section 308 has a lower material thickness than its adjacent sections. Thus, the bearing section 308 has a higher flexibility than its adjacent sections. Accordingly, the rotation axis 309 is in the bearing section 308.

Alternatively, it is also possible that the height wedge 300 comprises multiple portions, e.g two parts - a lower portion and an upper portion (fig. 15B). To enable the setting of lordosis, the upper portion can be lifted away from the lower portion.

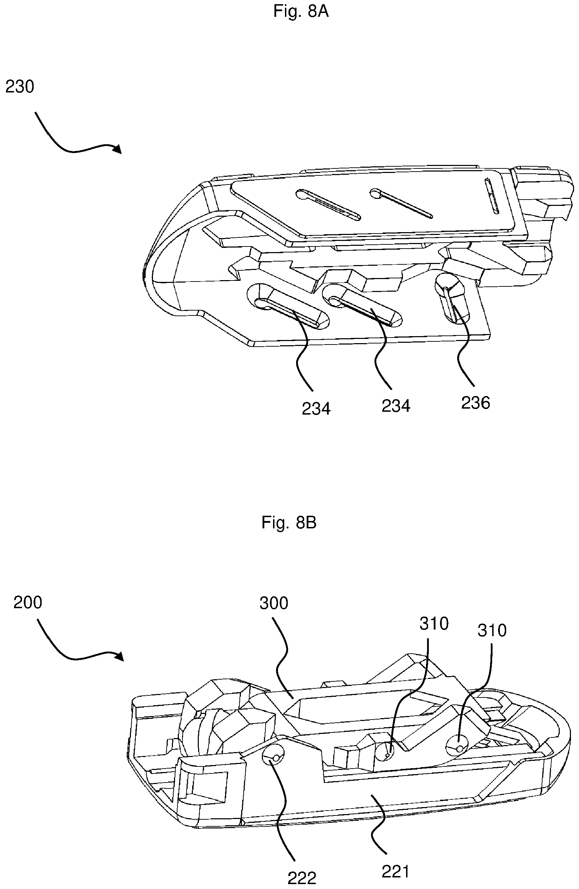

In figs. 8A, 8B it can be seen that the height wedge 300 comprises a connector portion 310 supporting a pullback of the height wedge 300 and a parallel lifting of the moving plate 230 simultaneously. The direction of action of such connector portion 310 is between vertical and horizontal but not equal to these limitations. The moving plate 230 also comprises a connector portion 234. The connector portion 310 of the height wedge 300 and the connector portion 234 of the moving plate 230 are configured to interact in such a way that a safe lifting of the moving plate 230 is supported. Therefore, one connector portion 234, 310, namely the connector portion 310 of the height wedge 300, is a protrusion like a conical pin while the other connector portion 234, 310, namely the connector portion 234 of the moving plate 230, is a conical groove. Thus, smooth sliding of the connector portion 310 in the connector portion 234 is enabled. Further, the moving plate 230 is thus fixed to the height wedge 300 and to the base plate 221 accordingly, preventing the moving plate 230 from unwanted detachment from the base plate 221. This is especially useful to enable a gentle insertion step of the cage during the surgical procedure, because now only a small insertion hole has to be dig into the remaining intervertebral disc.

In figs. 8A and 8B it can be seen that the height wedge 300 comprises multiple connector portions 310 and the moving plate 230 comprises also multiple connector portions 234 corresponding to the connector portion 310 of the height wedge 300.

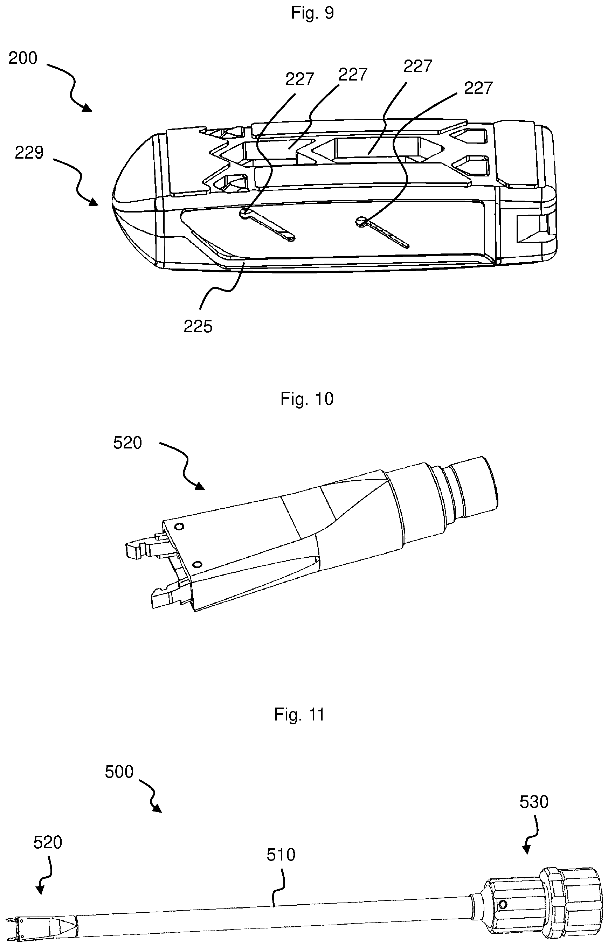

In fig. 9 an alternative to the vertical connector portion 222, 236 is displayed. This is a connection spring 225 comprised within the cage 200 which is configured to connect the moving plate 230 to the base plate 221. The connection spring 225 is configured to enable vertical, or rather parallel lifting of the moving plate 230 as well as its tilting to apply lordosis.

In fig. 15 it can be seen, that the lordosis wedge 400 also comprises a connector portion 410 configured as a bulge and the base plate 221 comprises a horizontal sliding mean 224 corresponding thereto, e.g. a groove (fig. 15C). The horizontal sliding mean 224 of the base plate 221 is horizontally aligned. Thus, horizontal movement of the lordosis wedge 400 is enabled, while movement away from this direction can be prevented, especially unwanted tilting away of the lordosis wedge 400 or the height wedge 300 connected thereto from the base plate 221 can be prevented.

In fig. 15B it can be seen, that the height wedge 300 also comprises a connector portion 310 configured as a bulge and the base plate 221 comprises a horizontal sliding mean 224 corresponding thereto, e.g. a groove (fig. 15C). Preferably, the horizontal sliding mean 224 of the base plate 221 corresponding to the connector portion 410 of the lordosis wedge 400 corresponds to the connector portion 310 of the height wedge 300 too (fig. 15C).

In fig. 9, an exhaust opening 227 comprised within the cage 200 is displayed. Thus, metal powder 105 remaining in the cage after 3D printing can be extracted. The exhaust opening 227 is positioned on a lateral surface of the moving plate 230.

In fig. 9, an insertion tip 229 comprised within the cage is displayed.

In fig. 23 a rough structure 250 on the outer, horizontal surface of the cage 200 is displayed.

In fig. 6, an instrument 500 for adjusting the cage 200 is displayed. It comprises an adjustment handle 540 and preferably a locking rod 570. The instrument 500 comprises two locking rods 570 each lockable to a wedge (figs. 5B, 19A, 19B). Accordingly, one locking rod 570 is a height locking rod 570 that locks to the height wedge 300 while the other locking rod 570 is a lordosis locking rod 570 that locks to the lordosis wedge 400. The locking rods 570 can be positioned side by side (fig. 19A). It is also possible that one locking rod 570 is positioned inside the other locking rod 570 (fig. 5B, fig. 19B). Accordingly, the inner locking rod 570 has a smaller diameter than the outer locking rod 570. Each locking rod 570 comprises a locking section 572. The locking section 572 is shaped to fit the corresponding locking receiver 316, 416 (fig. 5B). Preferably, the locking rods 570 are configured to be rotatable between an insertion position and a locking position.

The adjustment handle 540 comprises at least two adjustment means 550 (fig. 6). Each adjustment mean 550 corresponds to one locking rod 570 and is connected thereto in such a way that with the adjustment mean 550 translational movements of the locking rod 570 can be initiated (figs. 24A, 24B). The adjustment handle 540 further comprises at least two transmissions 560, each of which is connected to one corresponding adjustment mean 550 and one corresponding locking rod 570. Thus, the adjustment means 550 can be moved in an ergonomic way.

As displayed in fig. 25C, the adjustment handle 540 further comprises a guiding rod 546 configured to guide the translational movement of an adjustment mean 550, or the adjustment means 550. Thus, unwanted chocking of the locking rods 570 within the shaft 510 can be prevented. The adjustment handle 540 comprises two guiding rods 546. The adjustment handle 540 comprises a recess 548, configured to take up the guiding rod 546, or guiding rods 546 (fig. 25C). Thus, very precise guiding can be enabled.

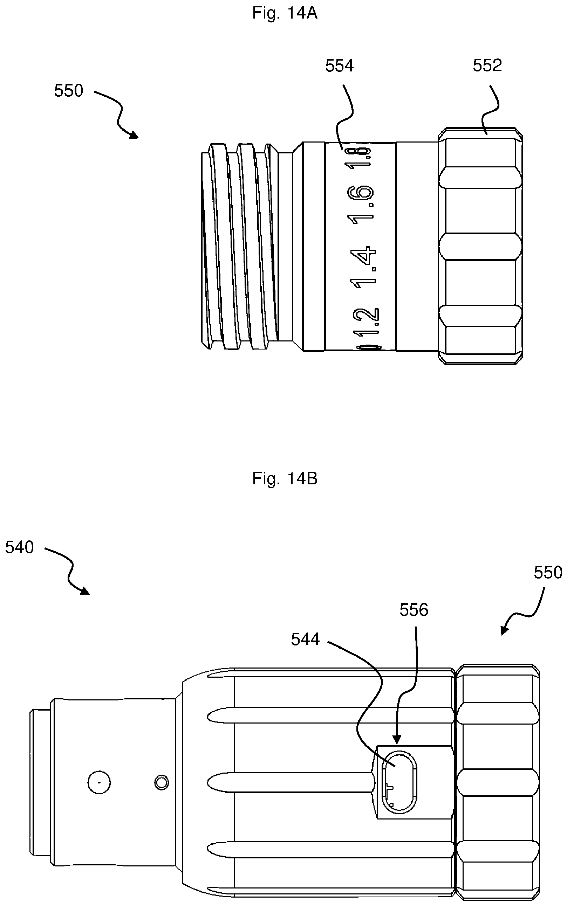

As can be seen in figs. 6, 14A, 14B, the adjustment handle 540 comprises a position indicator 542 configured to indicate the position of the locking rod 570, or locking rods 570. The adjustment handle 540 comprises a specific position indicator 542 for each locking rod 570, namely two position indicators 542 each for one of the two locking rods 570. The adjustment mean 550 comprises a manipulation portion 552 configured to manipulate the adjustment mean 550, namely to rotate it, and an indication portion 554 configured to indicate the position of the locking rod 570 connected to the adjustment mean 550. The adjustment handle 540 comprises an indication recess 544 configured to reveal the indication portion 554. The indication portion 554 and the indication recess 544 form a position display 556.

As can be seen in figs. 6, 10, the instrument 500 comprises a fixation device 520 configured to fix the instrument 500 with the cage. The fixation device 520 can be in an open and in a fixed position. The instrument 500 further comprises a fixation handle 530 configured to put the fixation device 520 in the open and in the fixed position (fig. 6).

As can be seen in fig. 11, the adjustment handle 540 is configured to get detached from the rest of the instrument 500. Thus, it is enabled that during surgical procedure and after adjusting the cage, the instrument 500 can be used for filling in the bone graft substitute. For this purpose, the shaft 510 is used as a cannula. This has the advantage that the bone graft substitute can be filled into the cage very precise. Additionally, time-consuming searching of the attachment point for filling in the bone graft substitute can be prevented.

As can be seen in fig. 6, the instrument 500 comprises a rotation mean 580, with which the locking rods 570 can be rotated. The instrument 500 comprises a fixation slot 582, with which the rotation mean 580 can be fixed in a specific position, e.g. in the locking position or in the insertion position. The rotation mean 580 can be configured as a rotation pin.

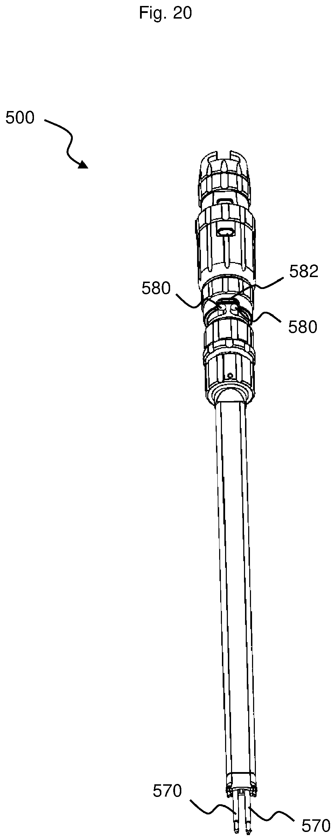

If the instrument 500 comprises two locking rods 570 positioned side by side, the instrument 500 comprises two rotation means 580, each connected to one locking rod 570 (fig. 20). The two locking rods 570 are fixed in the same fixation slot 582. To enable separate movement of the two rotation means 580, one can be configured to be rotatable into one direction (e.g. clockwise) while the other can be configured to be rotatable in the opposite direction (e.g. counterclockwise).

As can be seen in fig. 6, the instrument 500 further comprises a shaft 510 (fig. 6). The shaft 510 is connected with the adjustment handle 540. The shaft 510 is configured to receive and guide the locking rod 570. Thus, the locking rod 570 can be stabilized and protected from damage.

### Reference sign list

- 100: 3D printer
- 105: Metal powder
- 110: Substrate plate
- 120: Indexing table
- 130: Chamber
- 140: EDM configuration
- 142: Basing point
- 144: Wire
- 200: Adjustable intervertebral cage
- 202: Parallel sliding surface
- 210: Interface
- 215: Base
- 217: Twist lock (of the base)
- 221: Base plate
- 222: Connector portion
- 224: Horizontal sliding mean (of the base plate)
- 225: Connection spring
- 227: Exhaust opening
- 229: Insertion tip
- 230: Moving plate
- 234: Connector portion
- 236: Connector portion
- 238: Slotted hole
- 240: Ramp (of the moving plate)
- 242: Groove
- 244: Ramp
- 250: Rough structure
- 260: Guiding rail
- 262: Joint pin
- 270: Latching nose (of base or moving plate)
- 272: Leaf spring
- 274: Reset nose (of the base or moving plate)
- 280: Latching screw
- 282: Threaded part
- 284: Fastener receptacle
- 286: Latching part
- 290: Traction bolt
- 300: Height wedge
- 302: Base section
- 304: Moving section
- 306: Ramp (of the height wedge)
- 308: Bearing section
- 309: Rotation axis
- 310: Connector portion
- 312: Runner
- 314: Mating feature (for the height wedge)
- 316: Locking receiver (of the height wedge)
- 318: Latching nose (of the height wedge)
- 340: Twist lock (of the height wedge)
- 350: Reset nose (of the height wedge)
- 370: Cutting slot
- 380: Thread
- 390: Interacting means
- 395: Bore
- 400: Lordosis wedge
- 406: Ramp (of the lordosis wedge)
- 410: Connector portion
- 414: Mating feature (for the lordosis wedge)
- 416: Locking receiver (of the lordosis wedge)
- 420: Recess (of the lordosis wedge)
- 422: Latching recess
- 500: Instrument
- 510: Shaft
- 520: Fixation device
- 530: Fixation handle
- 540: Adjustment handle
- 542: Position indicator
- 544: Indication recess
- 546: Guiding rod
- 548: Recess
- 550: Adjustment mean
- 552: Manipulation portion
- 554: Indication portion
- 556: Position display
- 560: Transmission
- 562: Internal thread
- 564: External thread
- 570: Locking rod
- 572: Locking section
- 574: Clicking mechanism
- 580: Rotation mean
- 582: Fixation slot

## Claims

1. A process for manufacturing an adjustable intervertebral cage (200) by 3D printing with a base plate (221), a moving plate (230), a height wedge (300) and a lordosis wedge (400) comprising the steps of:
i) Providing a 3D printer (100) with a substrate plate (110), and metal powder (105);
ii) Applying metal powder (105) to the substrate plate (110);
iii) Selectively melting the metal powder (105) on the substrate plate (110) according to a layer of the adjustable intervertebral cage (200), wherein the layer of the base plate (221), the layer of the moving plate (230), the layer of the height wedge (300) and the layer of the lordosis wedge (400) are interlaced; and
iv) Sequentially repeating steps ii) and iii) layer by layer until the adjustable intervertebral cage (200) is fully manufactured, wherein all layers of the base plate (221), all layers of the moving plate (230), all layers of the height wedge (300) and all layers of the lordosis wedge (400) are each metallically bonded together.

2. The process according to claim 1, wherein after step iv), the remaining unmolten metal powder (105) on the substrate plate (110) is removed therefrom.

3. The process according to any preceding claim, wherein after step iv), the adjustable intervertebral cage (200) is cut from the substrate plate (110) by electrical discharge machining.

4. The process of any preceding claim, wherein after step iv), the adjustable intervertebral cage (200) is cut by electrical discharge machining.

5. The process according to claim 3 or 4, wherein the electrical discharge machining is performed with a wire (144).

6. The process of any preceding claim, wherein during steps iii) and iv), a rough structure (250) is printed onto at least a portion of the base plate (221) and/or the moving plate (230).

7. The process of any preceding claim, wherein during steps iii) and iv), the printed portion of the base plate (221), the moving plate (230), the height wedge (300) and the lordosis wedge (400) each are essentially spaced apart for at least 0.05 millimetres and at most 5 millimetres.

8. A 3D printed adjustable intervertebral cage (200), comprising
i) a moving plate (230) having at least one first connector portion (234, 236) integrally formed in the moving plate (230);
ii) a base plate (221) having at least one second connector portion (222) integrally formed in the base plate (221);
iii) a height wedge (300) having at least one third connector portion (310) integrally formed in the height wedge (300), and the height wedge (300) preferably having a moving section (304) and a base section (302), wherein the height wedge (300) is deformable so that the moving section (304) is inclineable with regard to the base section (302); and
iv) a lordosis wedge (400), moveably supported with respect to the height wedge (300) to control the inclination of the height wedge (300), the lordosis wedge (400) is preferably arranged in contact with the moving section (304) and the base section (302) of the height wedge (300) to control the inclination of the moving section (304),
wherein the connector portions (234, 236) of the moving plate (230), the height wedge (300) and the base plate (221) are interlaced within each other so as to maintain the structural stability of the adjustable intervertebral cage (200).

9. The adjustable intervertebral cage (200) according to claim 8, wherein the lordosis wedge (400) and the height wedge (300) comprise latching recesses (422), and wherein the adjustable intervertebral cage (200) comprises latching noses (270, 318) corresponding to the latching recesses (422) to prevent the adjustable intervertebral cage (200) from collapsing.

10. The adjustable intervertebral cage (200) according to claim 9, wherein the adjustable intervertebral cage (200) comprises at least one reset nose (274, 350) to unlock the latching recesses (422) and latching noses (270, 318) at least temporarily.

11. The adjustable intervertebral cage (200) according to any of claims 8 to 10, wherein the first and second connector portions (222, 234, 236) comprise a groove and/or a protrusion.

12. The adjustable intervertebral cage (200) according to any of claims 8 to 11, wherein one or more grooves comprise a printing recess which is formed deeper/wider than the rest of the groove.

13. The adjustable intervertebral cage (200) according to any of claims 8 to 12, wherein at least one of the first and second connector portions (222, 234, 236) comprise an exhaust opening (227) to outside of the moving plate (230) and/or base plate (221).

14. The adjustable intervertebral cage (200) according to any of claims 8 to 13, wherein at least the moving plate (230) and the height wedge (300) comprise ramps (240, 306) which serve to adjust the height distance between the moving plate (230) and the base plate (221).

15. The adjustable intervertebral cage (200) according to any of claims 8 to 14, wherein the height wedge (300) and the lordosis wedge (400) comprise locking receivers (316, 416) for receiving corresponding portions of an instrument (500) for relatively moving the height wedge (300) to the base plate (221) and the moving plate (230), and relatively moving the lordosis wedge (400) to the height wedge (300).

16. A 3D printed adjustable intervertebral cage (200), comprising
a first moving plate (230) having first adjustment means (240) and second adjustment means (144) and a second moving plate (230) having first adjustment means (240) and second adjustment means (144);
a height wedge (300) for adjusting the distance between the first and second moving plates (230), having first and second interacting means (306, 390) interacting with the first adjustment means (240) of the first and second moving plate (230), respectively; and
a lordosis wedge (400) for adjusting the inclination of the first and second moving plate (230), the lordosis wedge (400) comprises first and second interacting means (306, 390) interacting with the second adjustment means (144) of the first and second moving plate (230), respectively.

17. The adjustable intervertebral cage (200) according to claim 16, wherein the first adjustment means (240) of the first and second moving plate (230) and the first and second interacting means (306, 390) of the height wedge (300) are formed as grooves (242) and projections (390) and/or as ramps (306, 244).

18. The adjustable intervertebral cage (200) according to claim 17, wherein the grooves (242) are formed on both inner sides of the moving plates (230) and the projections (390) are formed on both outer sides of the height wedge (300).

19. The adjustable intervertebral cage (200) according to any of claims 16 to 18, wherein the second adjustment means (144) of the moveable plate (230) and the first and second interacting means (306, 390) of the lordosis wedge (400) are formed as ramps (240, 406).

20. The adjustable intervertebral cage (200) according to any of claims 16 to 19, wherein the height wedge (300) and/or the lordosis wedge (400) comprise a bore (395) for introducing an instrument (500).

21. The adjustable intervertebral cage (200) according to any of claims 16 to 20, wherein a first traction bolt (290) is arranged within the lordosis wedge (400) and the height wedge (300) to fixedly connect them and a second traction bolt (290) is arranged within the height wedge (300) and the moving plates (230) to fixedly connect the height wedge (300) with the moving plates (230).

22. A 3D printed adjustable intervertebral cage (200), comprising
a moving plate (230) having first adjustment means;
a base plate (221) rotatably connected with at least the moving plate (230), preferably on a rotation axis parallel to and on the long side of the adjustable intervertebral cage (200);
a height wedge (300) for adjusting the distance between the moving plate (240) and the base plate (221), the height wedge (300) comprises a moving section (304) and a base section (302) which are rotatably connected to each other, preferably on the same rotation axis as the rotation axis of the moving plate (230) and the base plate (221), wherein the height wedge (300) further comprises interacting means for interacting with the adjustment means of the moving plate (230) to adjust said distance and second adjustment means; and
a lordosis wedge (400) for adjusting the inclination of the moving plate (230), the lordosis wedge (400) is moveable relative to the height wedge (300) and comprises second interacting means interacting with the second adjustment means of the height wedge (300), to adjust the inclination of the moving plate (230) by rotating the moving section (304) of the height wedge (300).

23. The adjustable intervertebral cage (200) according to claim 22, wherein the adjustment means and the interacting means are formed as ramps (240, 316, 416).

24. The adjustable intervertebral cage (200) according to claim 22 or 23, further comprising latching screws (280), wherein a first latching screw (280) fixes the base section of the height wedge (300) to the base plate (221) in at least one translational direction and a second latching screw (280) fixes the base section of the height wedge (300) to the lordosis wedge (400) in at least one translational direction.

25. The adjustable intervertebral cage (200) according to claim 24, wherein the latching screws (280) comprise a head with hook elements and a pin with hook elements that interact with corresponding hook elements on the base plate (221), the lordosis wedge (400) and the height wedge (300).

26. The adjustable intervertebral cage (200) according to any of claims 22 to 25, wherein the lordosis wedge (400) and the height wedge (300) comprise accommodation portions for interaction with an instrument (500).

27. An instrument (500) for adjusting an adjustable intervertebral cage (200) with a height wedge (300) and a lordosis wedge (400) comprising:
i) a fixation device (520) shiftable into an fixed position and configured to be fixable with the adjustable intervertebral cage (200);
ii) a first locking rod (570) configured to interlock with the height wedge (300) and/or the lordosis wedge (400), and configured to transmit its movement into a movement of the height wedge (300) and/or the lordosis wedge (400);
iii) a fixation handle (530) configured to shift the fixation device (520) into the fixed position; and
iv) an adjustment handle (540) comprising a first adjustment mean (550), wherein the first adjustment mean (550) is configured to move the first locking rod (570).

28. The instrument (500) according to claim 27, wherein the instrument (500) comprises a second locking rod (570) and the adjustment handle (540) comprises a second adjustment mean (550) configured to move the second locking rod (570), wherein the first locking rod (570) is configured to interlock with the height wedge (300), and configured to transmit its movement into a movement of the height wedge (300), and wherein the second locking rod (570) is configured to interlock with the lordosis wedge (400), and configured to transmit its movement into a movement of the lordosis wedge (400).

29. The instrument (500) according to claim 27 or 28, wherein the adjustment handle (540) comprises a first transmission (560) and optionally a second transmission (560), wherein the first transmission (560) is connected to the first adjustment mean (550) and the first locking rod 570, and wherein optionally the second transmission (560) is connected to the second adjustment mean (550) and the second locking rod (570).

30. The instrument (500) according to any of claims 27 to 29, wherein the first adjustment handle (540) comprises a first position indicator (542) configured to indicate the position of the first locking rod (570), and wherein optionally the second adjustment handle (540) comprises a second position indicator (542) configured to indicate the position of the second locking rod (570).

31. The instrument (500) according to any of claims 27 to 30, wherein the instrument (500) comprises a shaft (510) configured to cover the first locking rod (570) and/or the second locking rod (570).

32. The instrument (500) according to any of claims 27 to 31, wherein the instrument (500) comprises a rotation mean (580) connected to the first locking rod (570) and/or optionally the second locking rod (570), and configured to apply a rotational movement to the first locking rod (570) and/or optionally the second locking rod (570).
